(19) Europäisches Patentamt
European Patent Office
Office européen des brevets

(11) **EP 2 511 689 A2**

(12) **EUROPÄISCHE PATENTANMELDUNG**

(43) Veröffentlichungstag:
**17.10.2012 Patentblatt 2012/42**

(51) Int Cl.:
***G01N 5/00*** *(2006.01)* ***G01N 15/02*** *(2006.01)*
***B08B 9/055*** *(2006.01)*

(21) Anmeldenummer: **12160104.1**

(22) Anmeldetag: **19.03.2012**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **13.04.2011 DE 102011007309**

(71) Anmelder: **Krones AG**
**93073 Neutraubling (DE)**

(72) Erfinder: **Scheuren, Hans**
**93055 Regensburg (DE)**

(74) Vertreter: **Grünecker, Kinkeldey,**
**Stockmair & Schwanhäusser**
**Leopoldstrasse 4**
**80802 München (DE)**

(54) **Verfahren und Vorrichtung zur Verschmutzungs- und Reinigungsvalidierung einer Anlage**

(57) Verfahren zum Bestimmen des Verschmutzungsgrades eines Untersuchungsobjekts wie etwa einer Anlage, insbesondere einer Anlage zur Abfüllung flüssiger Lebensmittel, oder eines Bauteils oder Teilabschnitts einer Anlage, insbesondere einem Rohr, mit dem Schritt des Messens einer Eigenschaftsverteilung eines ersten Untersuchungsmediums vor und nach dem Durchleiten durch das Untersuchungsobjekt.

FIG. 1A

EP 2 511 689 A2

## Masse Partikel

FIG. 1B

**Beschreibung**

Gegenstand der vorliegenden Erfindung ist ein Verfahren zum Bestimmen des Verschmutzungsgrades eines Untersuchungsobjekts, wie etwa einer Anlage oder von Teilen dieser Anlage, und ferner das Bestimmen des Reinigungsverhaltens des Untersuchungsobjekts, sowie eine Vorrichtung zum Durchführen des Verfahrens.

Die Beurteilung einer Produktionsanlage oder einzelner Teilabschnitte oder Bauteile, insbesondere Rohre, einer Anlage im Hinblick auf ihre Verschmutzungs- und Reinigbarkeit ist seit Jahren Gegenstand von Untersuchungen. Eine derartige Anlage oder Teilabschnitte, Bauteile, insbesondere Rohre, in einer Abfüllmaschine werden im Folgenden als "Untersuchungsobjekt" bezeichnet. Die Verschmutzbarkeit, also der Verschmutzungsgrad, sowie die Reinigbarkeit, also der Reinigungsgrad, eines solchen Untersuchungsobjekts kann im Folgenden auch als "Hygienestatus" abgekürzt werden. In der Regel soll die Bedienung einer Anlage, häufig auch einer bestehenden Anlage, um eine individuelle Reinigungsführung ergänzt werden. Hierzu wird erwartet, dass die Anlage eine Meldung oder ein Signal ausgeben kann, die die Notwendigkeit einer Reinigung anzeigt. Der hierfür nötige Grad der Verschmutzung, also die zu berücksichtigende Bewertungsgröße, ist bisher nicht definiert worden. Als Reaktion auf eine solche Meldung, respektive ein solches Signal, erfolgt der Reinigungsschritt. Mit dem Erreichen eines gewünschten Reinigungsgrades, welcher ebenfalls eine noch nicht definierte Bewertungsgröße darstellt, wird der neue, respektive der wiederhergestellte Zustand mit einer entsprechenden Meldung angezeigt und der Reinigungsprozess wird abgeschlossen. Anschließend kann der Produktionsprozess fortgesetzt werden.

Eine einfache Umsetzung eines solchen Verfahrens wäre ein optisches Signal. Beispielsweise könnte das Anzeigen für Verschmutzung und Reinigung mit dem Aufleuchten eines beispielsweise roten Lichtes (d. h. Anlage verschmutzt) und entsprechend eines grünen Lichtes (Anlage gereinigt) erreicht werden. Bisher ist ein solcher Prozess jedoch nicht umgesetzt worden. Dabei stellen sich bei den Untersuchungen die folgenden Fragen: Wie verschmutzt sich eine Anlage? Wie reinigt sich eine Anlage? Wie sind beide Vorgänge, die Verschmutzung wie auch die Reinigung zu erfassen und zu standardisieren? Eine Antwort auf die ersten beiden Fragen ist häufig sehr schwierig. Dabei kann es aber möglich sein, ein Optimum einer Anlage in Bezug auf ein Verschmutzungs- und Reinigungsverhalten zu formulieren. Beispielsweise kann eine solche Anlage schwer zu verschmutzen sein. Ein solche Anlage würde hohe Standzeiten aufweisen und entsprechend kurze Reinigungszeiten. Die Effizienz einer solchen Anlage wäre sehr hoch. Umgekehrt könnte eine Anlage geringe Standzeiten und entsprechend hohe Reinigungszeiten aufweisen. Die Wirtschaftlichkeit einer derartigen Anlage im Vergleich zur vorherigen Anlage wäre dabei sehr gering, wobei es sich versteht, dass davon ausgegangen werden soll, dass beide Anlagen ähnliche Verwendung haben sollen.

Verschmutzungen von oben skizzierten Anlagen, insbesondere Anlagen zur Abfüllung flüssiger Lebensmittel, beinhalten häufig ein Verschmutzen durch Partikel. Es stellt sich daher die Frage, inwieweit auf der Basis von Partikeltechnologien Verschmutzung und Reinigung einer Anlage betrachtet werden können. Die DE 10 2009 009426 A1 beschreibt ein Messverfahren und eine Messvorrichtung zur Bestimmung von Eigenschaften eines mit Partikeln beladenen Fluidstroms. Dabei wird aus der Kenntnis verschiedener Partikeleigenschaften, wie Dichte und Form, die Geschwindigkeit des Trägerfluids berechnet und schließlich auf die Geschwindigkeit eines Partikels oder von Partikeln in strömendem Medium berechnet. Entsprechend wird ein Strömungsrohr mit sensorischer Ausstattung verwendet.

Die WO 98/00694 beschreibt die Simulation des Verhaltens eines bestimmten Partikels durch einen Ersatz dieses Partikels durch ein analoges, vergleichbares und insbesondere messbares Partikels (das also nicht dem speziell betrachteten Partikel entspricht), wobei bestimmte Eigenschaften, wie Form oder Größe, denen des gewünschten Partikel entsprechen. Dabei werden ein oder mehrere bestimmte definierte Partikel in ihrer Bewegung erfasst und dementsprechend in einem Prozess, wie der Produktsterilisation oder Pasteurisation, betrachtet. Dabei wird insbesondere die Verweilzeit eines Partikels in der Anlage betrachtet. Dabei ist jedoch zu berücksichtigen, dass es sich um ein analoges Teilchen handelt, was in einer Simulation betrachtet wird.

Ein klassisches Kontrollelement im Hinblick auf die mikrobiologische Analyse von Produkt und Spülwasserproben ist die mikrobiologische Analyse in der Lebensmittelindustrie. Beispielsweise kann ein zu prüfendes Bauteil mit einer Mikroorganismuslösung verschmutzt, dann gereinigt, und anschließend mit einem Indikator hinsichtlich verbliebener Verunreinigungen detektiert und bewertet werden. Das Verfahren beinhaltet jedoch die Zerstörung des zu prüfenden Bauteils, eine Gesamtanlage kann damit nicht beurteilt werden.

Verfahrenstechnische Ansätze versuchen ferner, Kennzahlen bezüglich Stofftransport von Schmutzpartikeln aus den Grenzbereichen eines Rohres zu berechnen. Dabei werden Gleichungen für Wärme- und Stofftransport verwendet. Hier wird auf die Vorausberechnung oder die Simulation des Reinigungsverhaltens abgezielt. Eine praktische Überprüfung ist jedoch mit diesem Ansatz nicht gegeben. Auch kann eine Verschmutzung und eine damit verbundene Standzeit in der Anlage nicht berechnet werden. Die verschiedenen Verfahren und Methoden liefern Aussagen zur Effizienzsteigerung und Überwachung eines Reinigungsprozesses.

Jedoch sind diese Verfahren und Methoden aufwändig und in der Regel nicht geeignet, insbesondere eine Gesamtanlage hinsichtlich des Verschmutzungs- und Reinigungsverhaltens in der Gesamtheit zu erfassen.

Angesichts der oben zitierten Probleme und angesichts des skizzierten Stands der Technik stellt sich die Aufgabe auf der Basis von Partikeltechnologien ein Bewertungsmaßstab und ein Bewertungswerkzeug zu erstellen, der es erlaubt, ein Untersuchungsobjekt, wie etwa eine Anlage oder eine Rohrleitung, im Hinblick auf das Verschmutzungs- und Reinigungsverhalten zu quantifizieren.

Diese Aufgabe wird durch ein Verfahren gemäß dem Patentanspruch 1 sowie einer Vorrichtung gemäß dem Patentanspruch 17 gelöst.

Das erfindungsgemäße Verfahren umfasst ein Verfahren zum Bestimmen des Verschmutzungsgrades eines Untersuchungsobjekts, wie etwa einer Anlage, insbesondere einer Anlage zur Abfüllung flüssiger Lebensmittel, oder eines Bauteils oder Teilabschnitts einer Anlage, insbesondere einem Rohr wobei eine Eigenschaftsverteilung eines ersten Untersuchungsmediums vor und nach dem Durchleiten durch das Untersuchungsobjekt gemessen wird. Hierbei soll eine Eigenschaftsverteilung insbesondere im Hinblick auf Eigenschaften von Teilchen, insbesondere Partikeln im ersten Untersuchungsmedium, verstanden werden. Die Eigenschaften sind insbesondere Eigenschaften, wie die Größe der Teilchen, die in dem ersten Untersuchungsmedium auftreten. Weitere zu berücksichtigende Eigenschaften eines Partikels können die Anzahl, die Länge, die Oberfläche und das Volumen des Partikels sein. Weiterhin können auch die Farbe des Untersuchungsmediums, insbesondere mit den Partikeln darin, im Hinblick auf Lichtbrechung, Beugung und Absorption betrachtet werden.

Dabei kann insbesondere das Verhalten des Untersuchungsobjekts im Hinblick auf ein durchgeleitetes Untersuchungsmedium betrachtet werden. Dabei wird insbesondere der Trenneinfluss des Untersuchungsobjekts, wie etwa der Anlage, auf das durchgeleitete Untersuchungsmedium betrachtet. Also wird das Untersuchungsmedium vor und nach dem Durchleiten des Untersuchungsobjekts betrachtet. Dabei ist der gewählte Ansatz mechanisch-verfahrenstechnisch und dabei ist die Reproduzierbarkeit des Ansatzes sehr hoch. Das Verfahren berücksichtigt an dieser Stelle keine mikrobiologischen Standards zur Beurteilung eines Hygienestatus, welcher aber, sofern sie vorliegen, noch zu diesem Verfahren hinzugenommen werden können.

Das erste Untersuchungsmedium kann typischerweise ein erstes Fluid umfassen. Dabei kann das erste Fluid beispielsweise ein flüssiges Lebensmittel umfassen. Das erste Fluid kann so Wasser umfassen. Typischerweise kann das erste Untersuchungsmedium einen Feststoff, und insbesondere eine Mischung, wie etwa eine Suspension des ersten Fluids und des Feststoffs, umfassen. Typischerweise umfasst der Feststoff kleine Partikel, die in der Suspension schweben, sofern diese gerührt wird, und langsam sedimentieren, wenn die Suspension still steht.

Im Hinblick auf den Einsatz für Abfüllanlagen für Lebensmittel, insbesondere Säfte oder Flüssigkeiten, denen Zusatzstoffe zugesetzt sind, können die Partikel solche Feststoffe umfassen, die im Lebensmittel enthalten sind, z. B. Fruchtfleisch, oder die dem Lebensmittel zugesetzt worden sind. Ebenso können die Partikel sandartige Partikel, insbesondere Quarzsand, umfassen. Hierdurch ist es auch möglich, etwa eine Kurzzeiterhitzungsanlage für Milchprodukte im Hinblick auf deren Hygienestatus zu beurteilen, in dem das Aggregat von einer Quarzsand-Milch-Suspension durchströmt wird.

In dem erfindungsgemäßen Verfahren, wie oben beschrieben, kann die Eigenschaftsverteilung des ersten Untersuchungsmediums, insbesondere die Partikelgrößenverteilung und/oder die Änderung der Partikelgrößenverteilung vor und nach dem Durchleiten durch das Untersuchungsobjekt umfassen.

Ferner kann das erfindungsgemäße Verfahren zusätzlich beim Messen der Eigenschaftsverteilung des ersten Untersuchungsmediums die Massenänderung, insbesondere im Hinblick auf prozentualen Massenverlust oder Massenzuwachs, betrachten. Dabei versteht sich, dass vor und nach dem Durchleiten des Untersuchungsmediums durch das Untersuchungsobjekt ein geeignetes Testvolumen betrachtet werden kann, beispielsweise einen Liter (1 l) oder eine größere Volumeneinheit. Ferner versteht, dass in dem oben geschilderten Verfahren der Einfluss einer Fließgeschwindigkeit des Untersuchungsmediums durch das Untersuchungsobjekts möglichst gering gehalten werden sollte. Das heißt, das Untersuchungsmedium sollte beispielsweise nur mit schwachem Druck durch die Anlage gepumpt werden. Die Pumpgeschwindigkeit sollte dabei typischerweise nicht verändert werden, bis ein Messwert erzielt worden ist.

Das erfindungsgemäße Verfahren kann ferner das Bestimmen des Reinigungsverhaltens des Untersuchungsobjekts nach dem Bestimmen des Verschmutzungsgrades wie oben beschrieben umfassen, bei mit dem ersten Untersuchungsmedium gefüllten Untersuchungsobjekt. Nach dem Bestimmen des Verschmutzungsgrades ist in der Regel das Untersuchungsobjekt mit dem ersten Untersuchungsmedium gefüllt. Somit ist typischerweise vor dem Beginn des Bestimmens des Reinigungsverhaltens eine Eigenschaftsverteilung des ersten Untersuchungsmediums, welches durch das Untersuchungsobjekt geleitet wurde, bekannt. Zumindest ist typischerweise das Untersuchungsobjekt mit dem ersten Untersuchungsmedium gefüllt, bevor das Bestimmen des Reinigungsverhaltens erfolgt. Das Verfahren umfasst typischerweise nachfolgend den Schritt des Messens einer Eigenschaftsverteilung eines zweiten Untersuchungsmediums vor und nach dem Durchleiten durch das Untersuchungsobjekt und Vergleichen der Eigenschaftsverteilung des zweiten Untersuchungsmediums nach dem Durchleiten durch das Untersuchungsobjekt mit der Eigenschaftsverteilung vor dem Durchleiten durch das Untersuchungsobjekt und mit der Eigenschaftsverteilung des ersten Untersuchungsmediums. Dabei kann das zweite Untersuchungsmedium ein zweites Fluid umfassen, wobei insbesondere das zweite Fluid dem ersten Fluid entspricht, also beispielsweise für das erste und das zweite Untersuchungsmedium Wasser oder Milch als Fluid oder ein anderes Lebensmittel verwendet werden. Dabei enthält das zweite Untersuchungsmedium in der Regel vor dem Einleiten/Durchleiten durch das Untersuchungsobjekt keine Partikel, wie etwa Sand, wie oben beschrieben. Während des Durchleitens durch das Untersuchungsobjekt kann das zweite Untersuchungsmedium, also in der Regel das zweite Fluid, Partikel aufnehmen. Entsprechend dem erfindungsgemäßen Verfahren kann die Eigenschaftsverteilung des zweiten Untersuchungsmediums die Bestimmung der Partikelgrößenverteilung und/oder die Änderung der Partikelgrößenverteilung vor und nach dem Durchleiten umfassen.

Dabei kann beim Messen der Eigenschaftsverteilung des zweiten Untersuchungsmediums die Massenänderung, insbesondere der prozentuale Massenverlust oder Massenzuwachs hinsichtlich des zweiten Untersuchungsmediums, bestimmt werden. Dabei gilt es also, dass das zweite Untersuchungsmedium typischerweise beim Durchleiten durch das Untersuchungsobjekt dort Partikel aufnimmt. Diese Partikel stammen typischerweise aus der Bestimmung des Verschmutzungsgrades entsprechend des erfindungsgemäßen Verfahrens wie oben beschrieben.

Das Messen der Eigenschaftsverteilung, insbesondere der Partikelgrößenverteilung, kann mittels eines Laserbeugungsspektrometers erfolgen. Dabei kann anhand eines auf die Probe gerichteten Lasers und mit dem auf diese Art erzeugten Interferenzmuster die Partikelgrö-βenverteilung vor und nach dem Durchleiten durch das Untersuchungsobjekt gemessen werden. Weiterhin kann die Detektion der Massenänderung beispielsweise wenigstens zu Beginn und zum Ende des jeweiligen Messvorgangs erfolgen. Dadurch ist beispielsweise bekannt, wie groß der Massenverlust, also die Massenänderung während des Bestimmens des Verschmutzungsgrads bezüglich des ersten Untersuchungsmediums sein kann. Entsprechend kann betrachtet werden, inwieweit das zweite Untersuchungsmedium durch das Durchleiten durch das verschmutzte Untersuchungsobjekt Partikel aufnehmen kann und diese sich in einer Massenänderung nach dem Durchleiten des zweiten Untersuchungsmediums zeigen.

Ferner kann die Erfindung, wie oben beschrieben, eine Vorrichtung zum Durchführen des Verfahrens wie oben beschrieben umfassen. Diese Vorrichtung kann einen Aufgabe- und einen Ausgabebehälter umfassen. Diese können beispielsweise vom Untersuchungsobjekt getrennt sein. Ferner kann die Vorrichtung eine geeignete Förder- oder Transporteinrichtung, insbesondere Rohre, umfassen, um das erste und/oder das zweite Untersuchungsmedium aus dem Aufgabebehälter zu entnehmen und das erste oder das zweite Untersuchungsmedium durch das Untersuchungsobjekt durchzuleiten. Ferner kann die Fördervorrichtung ausgebildet sein, die Untersuchungsmedien nach dem Durchleiten durch das Untersuchungsobjekt auszugeben und in einem Ausgabebehälter aufzufangen. In dem Ausgabebehälter kann eine Detektionsvorrichtung, insbesondere ein Laserbeugungsspektrometer, ausgebildet sein zur Bestimmung der Eigenschaftsverteilung des ersten oder des zweiten Untersuchungsmediums. Es versteht sich, dass auch andere optische geeignete Vorrichtungen in der Vorrichtung vorgesehen sein können, welche die Eigenschaftsverteilung der Untersuchungsmedien bestimmen. Ferner kann der Aufgabe und/oder Ausgabebehälter mit einer Homogenisierungsvorrichtung, insbesondere einen Rührer, versehen sein, um die Mischung von Fluid und Partikel vor und nach dem Durchleiten durch die Anlage entsprechend zu durchmischen. Dabei ist klar, dass die Homogenisierung der Sedimentation im Aufgabe- und/oder Ausgabebehälter entgegenwirkt, so dass nur innerhalb des Untersuchungsobjekts eine Sedimentation möglich scheint. Ferner können zusätzlich in der Vorrichtung weitere Messvorrichtungen zur Messung von Temperatur, Druck, Partikelgrößen von Partikeln oder weitere Eigenschaftsverteilungen in den Aufgabe- und/oder Ausgabebehältern und im Untersuchungsobjekt selbst vorhanden sein, sofern auch spektroskopische Daten während des Durchleitens durch das Untersuchungsobjekt gewünscht sind. Ferner kann die erfindungsgemäße Vorrichtung mit einem Messsystem versehen sein, welches Laserbeugungsspektroskopie zur Bestimmung der Partikelgrößenverteilung und zur Massenbestimmung eingesetzt.

Zur Veranschaulichung wird nachfolgend das Beispiel einer idealen und einer realen hygienegerechten Rohrleitung beschrieben.

Ein ideales Rohr, d. h. ein Rohr, das im Wesentlichen gerade ist, selbstentleerend, polierte Innenoberflächen ohne Spalten und Kerben besitzt und keine Toträume oder zusätzliche Anbindungsstellen besitzt, soll durch eine Sand-Wasser-Suspension entsprechend der Erfindung untersucht werden. Ein solches Rohr beeinflusst idealerweise die Zusammensetzung der Sand-Wasser-Suspension zu keinem Zeitpunkt. Entsprechend ist die Partikelgrößenverteilung zu jedem Zeitpunkt und zu jedem Ort, unter Berücksichtigung der Strömungsform im Rohr, sowie Druck und Temperatur, konstant. Das bedeutet, dass im Wesentlichen die Gesamtmasse an Partikeln, die aufgegeben wird, auch wieder am Ende des Rohrs aufgefangen wird, nachdem sie durch das Rohr durchgeleitet worden sind. Damit ist die mechanische Verschmutzbarkeit dieser Anlage sehr gering, idealerweise sogar 0, das Rohr ist also nicht zu verunreinigen.

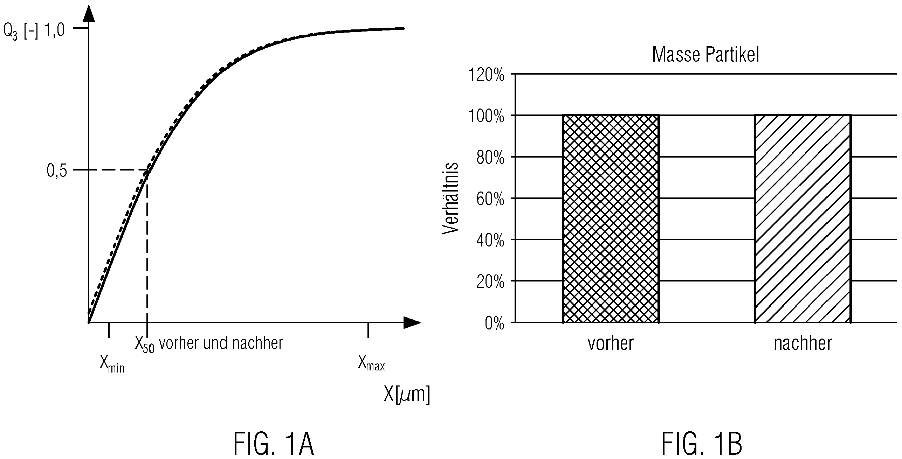

Die Fig. 1 und 1A zeigen die Untersuchung eines derartigen Rohres. Fig. 1A zeigt Partikelgrößenverteilung vor dem Durchleiten der Sand-Wasser-Suspension durch das Rohr als gepunktete Linie und die Partikelgrößenverteilung nach dem Durchleiten der Sand-Wasser-Suspension durch das Rohr. Fig. 1B zeigt ferner die Massenänderung.

Fig. 1A zeigt auf der Ordinate D minimalen Durchmesser der in der Suspension, also dem Sand-Wasser-Gemisch, gemessenen Durchmesser der Teilchen $x_{min}$. Ferner zeigt die Abszisse den entsprechenden maximalen Durchmesser der untersuchten Partikel der Suspension $x_{max}$. Dabei kann x zwischen $x_{min}$ und $x_{max}$ liegen und hat typischerweise die Größenordnung von einigen Mikrometern. Ferner ist der $x_{50}$-Wert eingezeichnet. Dieses ist der Median, d. h. die Hälfte aller Partikel, bezogen auf eine bestimmte Mengenart, ist größer oder kleiner als dieser Wert. Auf der Ordinate von Fig. 1A ist die Größe $Q_3$ aufgetragen. Dabei handelt es sich um die Verteilungssumme $Q_r (x_i)$, mit

r = 3, die sich auf das Volumen, respektive die Masse, bezieht. Dabei ist $Q_3(x_i)$ für einen betrachteten Durchmesser $x_i$ gegeben durch

$$Q_3(x_i) = \frac{Menge\_aller\_Partikel\_mit\_x \leq x_i}{Gesamtmenge\_aller\_Partikel}.$$

In Fig. 1A liegen die gepunktete und die gestrichene Kurve übereinander. Die Darstellung verdeutlicht also, dass alle Partikel jeder Größe, die in das Rohr gelangen, es auch wieder verlassen. Die Zusammensetzung zu den dargestellten Zeitpunkten "Vorher" und "Nachher" wird dadurch nicht verändert. Der $x_{50}$-Wert ist unverändert. Entsprechend zeigt die Fig. 1B die Massenänderung durch das betrachtete ideale Rohr. Diesem fiktiven Beispiel ist die Massenänderung vor und nach dem Durchleiten durch das ideale Rohr gleich. Das heißt, es erfolgt gar keine Massenveränderung und 100 % der betrachteten Teilchen ist wieder aus dem Rohr geleitet worden. Auch hier wieder auf entsprechende geeignete Normierung auf ein Testvolumen, z. B. 1 Liter oder größere oder kleinere Volumina, zu achten.

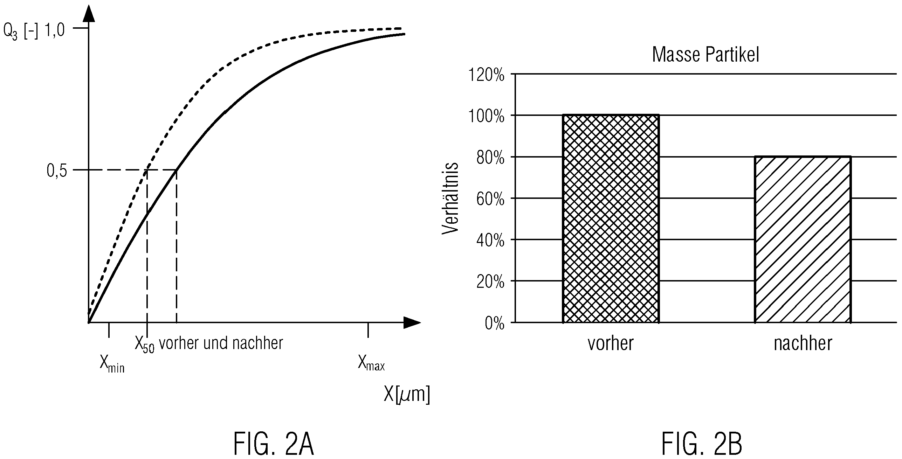

Ferner soll nun ein nicht ideales, also reales, Rohr betrachtet werden. Ein solches Rohr kann beispielsweise gekrümmt sein, insbesondere im Inneren eine raue Oberfläche mit Spalten und Toträumen aufweisen oder gar Anschlussstellen für weitere Rohre aufweisen. Entsprechend wird die Suspensionszusammensetzung beim Durchleiten der Quarz-Sand-Suspension zu jedem Zeitpunkt beeinflusst. Es kann eine Trennung von einzelnen Partikelgrößen erfolgen, so dass die Partikelgrößenverteilung über den Versuchszeitraum variieren kann. Ist der Versuch zeitlich ausreichend lang dimensioniert, dann ist die maximale Verschmutzung der Anlage, also der Verschmutzungsgrad, erreicht, wenn die Partikelgrößenverteilung der durchströmenden Suspension sich nicht mehr verändert. Dieses kann auch als Validierung der Verschmutzbarkeit oder Referenzpunkt für die Verschmutzbarkeit bezeichnet werden. Die Gesamtmasse an Partikeln, die aufgegeben wird, wird nicht wiederaufgefangen. Entsprechend zeigt Fig. 2 einen Unterschied zwischen den Massenänderungen vor und nach dem Durchleiten der Suspension durch das reale Rohr. Ebenso zeigt Fig. 2A eine Verschiebung der gepunkteten gegenüber der gestrichenen Linie und eine Veränderung des entsprechenden $x_{50}$-Wertes. Fig. 2A und 2B zeigen ansonsten die gleichen Größen wie die entsprechenden Fig. 1A und 1B. Der $x_{50}$-Wert ist in Fig. 2A erhöht. Daraus lässt sich ablesen, dass viele, insbesondere kleine, Partikel in der Anlage verbleiben. Entsprechend verringert sich die Gesamtmasse an Partikeln.

Als weiterer Schritt wird nun das Bestimmen des Reinigungsverhaltens der betrachteten Rohre diskutiert. Da für beide Rohre eine Sand-Wasser-Suspension zum Durchleiten verwendet wurde, wird zur Bestimmung des Reinigungsverhaltens nun Wasser durch die Anlage, d. h. die Rohre, geleitet. Mit Austritt der Flüssigkeit, also des Wassers, das nun eventuell in den Rohren verbliebene Partikel aufgenommen hat, wird nun die Partikelgrößenverteilung erneut detektiert und die Gesamtmasse an Partikeln erfasst. Treten keine Änderungen mehr auf oder sind alle Partikel wieder aufgefangen, so gilt die Anlage gereinigt. Man kann dies auch als "Validierung der Reinigbarkeit der Anlage" bezeichnen. Die Änderung der Partikelgrößenverteilung, wobei die $Q_3$-Verteilung beispielhaft verwendet wurde, es aber möglich ist, eine andere Mengenart zu verwenden oder in eine Mengenart umzurechnen, beschreibt das Reinigungsverhalten der betrachteten Anlage, hier des betrachteten realen Rohrs. Verbleiben kleine Partikel in der Anlage und steigt dementsprechend der $x_{50}$-Wert, bezogen auf ein entsprechend zu definierenden Äquivalentfaktor, dann kann dies aus dem Vorhandensein vieler kleiner Spalte erklärt werden. Verändert sich dagegen der $x_{50}$-Wert kaum, und ist trotzdem es nicht möglich, die Masse an Partikeln wieder aus der Testanlage zurückzuhalten, dann kann dies bedeuten, dass große und kleine Spalten vorhanden sind, durch die selektiv sowohl kleine als auch große Partikel festgehalten werden.

Das entsprechend vorgestellte erfindungsgemäße Verfahren erlaubt es, den Hygienestatus einer Anlage zu beurteilen. Es ergibt sich sogar die Möglichkeit zwei Anlagen zu vergleichen. Das skizzierte Idealrohr kann als Optimum und damit als Referenzwert betrachtet werden. Bezogen auf diesen Standard können damit andere Rohre verglichen und klassifiziert werden. Eine entsprechende Standardisierung kann auf Anlagenteile oder ganze Anlagen übertragen werden. Damit können quantifizierbare Aussagen, wie etwa - rein fiktiv und beispielhaft - die Aussage, dass "ein untersuchtes Rohr beispielsweise fünfmal so leicht zu verschmutzen wie das Referenzrohr sei" getroffen werden.

Entsprechendes gilt für die Reinigung. Die Erfindung stellt den Vorteil bereit, dass auch komplexere Anlagen, insbesondere Verrohrungen, sowie ganze Maschinen auf diese Weise beurteilt werden können. Es ist sogar möglich, das Durchflussmedium von Wasser auf ein anderes flüssiges Lebensmittel zu ändern. Wichtig ist dabei, dass stets das Gesamtpartikelkollektiv betrachtet wird. Ein anderes Suspensionsmedium bewirkt andere Wechselwirkung und kann Veränderungen des entsprechenden Versuchsergebnisses bewirken, welches jedoch wieder entsprechend referenziert und normiert werden kann.

Der Erfindungsgegenstand wird anhand der nachfolgenden Zeichnung beispielhaft erläutert.

Es zeigen:

Fig. 1A    Übersicht Partikelgrößenverteilung für ein ideales Rohr.

Fig. 1B    Übersicht Massenänderung für ein ideales Rohr.

Fig. 2A    Partikelgrößenverteilung für ein reales Rohr.

Fig. 2B    Massenänderung für ein reales Rohr.

Fig. 3     Prinzipskizze für eine erfindungsgemäße Vorrichtung.

Fig. 4     eine Ausbildung einer erfindungsgemäßen Vorrichtung.

Fig. 5     Skizze des erfindungsgemäßen Verfahrens.

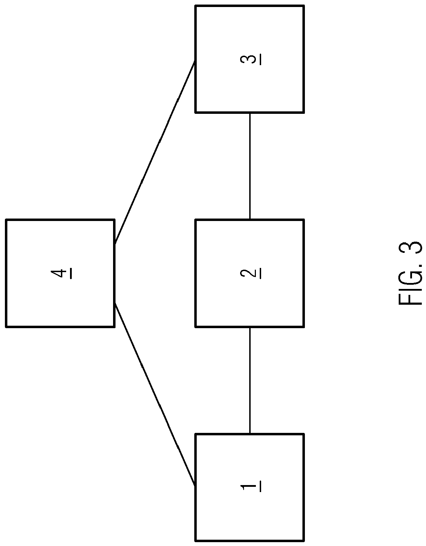

Die oben beschriebenen erfindungsgemäßen Ideen zur Bestimmung des Verschmutzungsgrades des idealen Rohres sowie eines realen Rohres und entsprechend eines Reinigungsgrades eines idealen Rohres wurden in den Fig. 1A, 1B, 2A und 2B dargestellt und diskutiert. Entsprechend den Figuren können Standards für Validierung festgelegt werden. Fig. 3 zeigt die Umsetzung des prinzipiellen Schemas zur diskontinuierlichen und/oder kontinuierlichen Betrachtungsweise des Verschmutzungsgrades und des Reinigungsverhaltens einer Anlage mit einer entsprechenden Vorrichtung. Fig. 3 zeigt einen Aufgabebehälter 1, einen Auffangbehälter 3 und ein Untersuchungsobjekt 2. Diese sind typischerweise in Reihe installiert. Die Aufgabe- und Auffangbehälter 1 und 3 sind beispielsweise vom Untersuchungsobjekt 2 getrennt installierbar. Zur Verbindung dieser Behälter mit dem Untersuchungsobjekt 2 können geeignete Rohre dienen. Aus dem Aufgabebehälter 1 wird ein Untersuchungsmedium, beispielsweise eine Sand-Wasser-Suspension, durch das Untersuchungsobjekt 2 geleitet oder gepumpt. Im Anschluss wird das Untersuchungsmedium, also die Suspension, in den Auffangbehälter 3 geführt. In definierten Zeitabständen wird die Partikelgrößenverteilung der Suspension mittels einer Detektionsvorrichtung 4 bestimmt. Wenigstens zu Beginn und zum Ende des Prozesses wird die Masse der aufgegebenen und der zurückerhaltenen Partikel, beispielsweise Sand, durch die Detektionsvorrichtung 4 gemessen.

Dabei kann die Bestimmung der Partikelgrößen und damit der Partikelgrößenverteilung in kontinuierlicher Form geschehen.

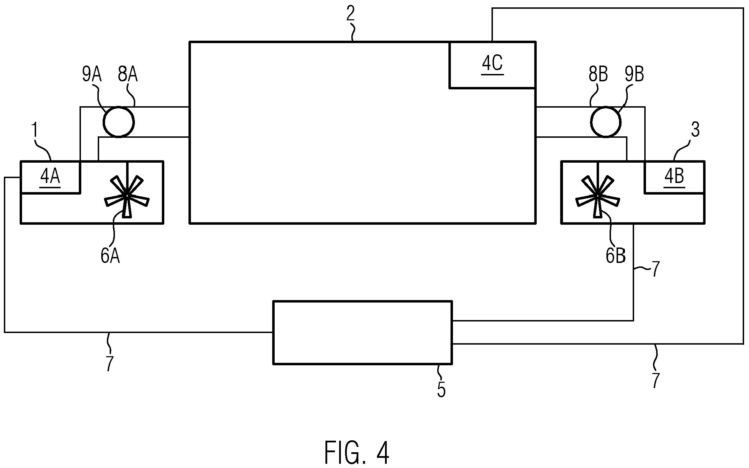

Fig. 4 zeigt eine Ausbildung entsprechend der vorliegenden Erfindung. Fig. 4 zeigt insbesondere einen Aufgabebehälter 1 und einen Auffangbehälter 3 sowie ein Untersuchungsobjekt 2. Fig. 4 zeigt, dass der Auffangbehälter 1 und der Ausgabebehälter 3 jeweils mit Rohren 8a und 8b mit dem Untersuchungsobjekt 2 verbunden sind. Das Untersuchungsobjekt 2 kann eine Anlage zur Behandlung oder Abfüllung flüssiger Lebensmittel sein. Die Rohre 8a und 8b können Pumpen 9a und 9b aufweisen, welche ein Untersuchungsmedium aus dem Aufgabebehälter 1 in das Untersuchungsobjekt 2 hinein leiten und aus dem Untersuchungsobjekt 2 in den Auffangbehälter 3 wieder herausleiten. Dabei ist typischerweise die Pumpgeschwindigkeit der Pumpen 9a und 9b konstant und gering. Der Aufgabebehälter 1 sowie der Auffangbehälter 3 umfassen ferner jeweils Vorrichtungen 6a und 6b, insbesondere Rührer, zur Homogenisierung des in den jeweiligen Behältern vorhandenen Untersuchungsmediums. Die Rührer 6a und 6b können also eine Sedimentation von Partikeln der in den Auffang/Aufgabebehältern vorhandenen Suspension verhindern. Fig. 4 zeigt ferner eine Steuereinheit 5, etwa eine CPU oder ein Computer, welcher über Steuerleitungen 7 mit Steuer-und/oder Messvorrichtungen 4a und 4b im Aufgabebehälter 1 und Auffangbehälter 3, respektive, verbunden ist. Dadurch können mit beispielsweise zwei Messsystemen 4a und 4b, welche Laserbeugungsspektrometer umfassen können, die Partikel in den Suspensionen im Aufgabebehälter 1 und Auffangbehälter 3 bestimmt werden. Also lassen sich die Partikelgrößenverteilung der Partikel bestimmen. Ferner zeigt Fig. 4 optional auch eine Messeinheit 4c, sofern es gewünscht ist, auch innerhalb des Untersuchungsobjekts 2 Daten über die Partikelgrößenverteilung der durchgeleiteten Suspension zu gewinnen. Ein derartiger Ansatz ist insbesondere für größere Anlagen möglich. Ebenso kann es möglich sein (hier nicht gezeigt), dass im Hinblick auf eine diskontinuierliche Form der Probennahme lediglich ein Messaggregat 4b in dem Auffangbehälter 3 vorhanden sein muss. Der Messschritt kann somit unabhängig vom Durchleiten der Suspension durch das Untersuchungsobjekt, und insbesondere nach dem Durchleiten durch das Untersuchungsobjekt erfolgen. Dabei ist es besonders vorteilhaft, wenn der Rührer 6b die im Auffangbehälter 3 aufgefangene Suspension homogenisiert.

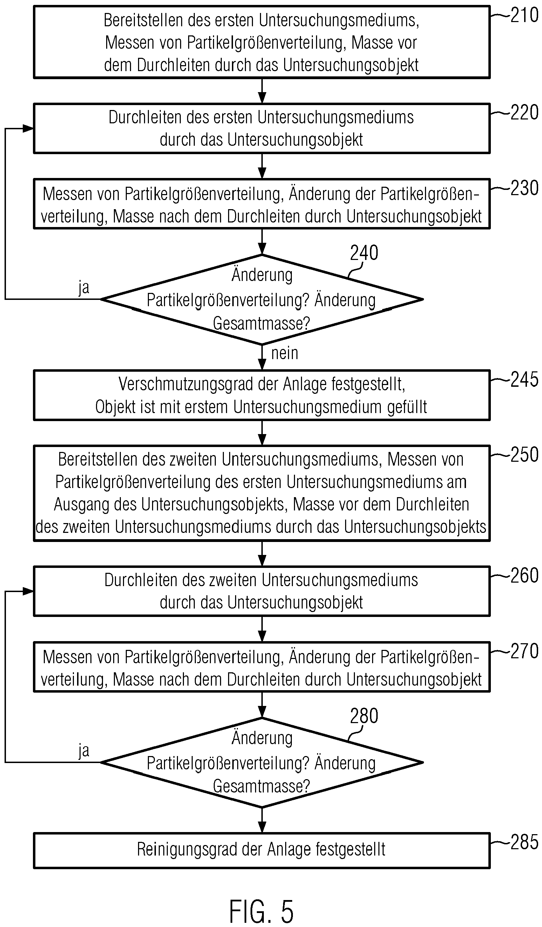

Fig. 5 zeigt beispielhaft ein Verfahren entsprechend der vorliegenden Erfindung. Im Schritt 210 erfolgen das Bereitstellen eines ersten Untersuchungsmediums, etwa einer Suspension von Sand und Wasser, sowie das Messen von einer Eigenschaftsverteilung, wie einer Partikelgrößenverteilung, vor dem Durchleiten durch das Untersuchungsobjekt. Entsprechend Fig. 4 kann solche Messung beispielsweise im Aufgabebehälter erfolgen. Im Schritt 220 erfolgt das Durchleiten des ersten Untersuchungsmediums durch das Untersuchungsobjekt. Im Schritt 230 erfolgt das Messen von einer Partikelgrößenverteilung, sowie der Änderung der Partikelgrößenverteilung gegenüber der im Schritt 210 gemessenen Partikelgrößenverteilung und ferner die Betrachtung der Gesamtmasse nach dem Durchleiten durch das Untersuchungsobjekt. Im Schritt 240 wird geprüft, ob eine Änderung der Partikelgrößenverteilung gegenüber dem ersten Messschritt erfolgt ist. Falls ja, wird mit dem Durchleiten des ersten Untersuchungsmediums fortgefahren, entsprechend dem Schritt 220. Dabei versteht sich, dass jeweils der letzte Wert der Partikelgrößenverteilung und der Gesamtmasse betrachtet wird, wenn der Schritt 240 erneut erreicht wird. Sofern im Schritt 240 keine Änderungen mehr auftreten, folgt Schritt 245, in dem der Verschmutzungsgrad der Anlage als "festgestellt" betrachtet wird.

Im Schritt 245 ist das Objekt aufgrund der vorangegangenen Schritte 210-240 mit dem Untersuchungsmedium, d. h. dem ersten Untersuchungsmedium, gefüllt. Sollten die expliziten Messschritte der vorangegangenen Schritte 210 - 240 nicht durchgeführt werden, so sollte wenigstens sicher gestellt werden, dass das Untersuchungsobjekt mit dem ersten Untersuchungsmedium, also etwa einer Sand-Wasser Suspension, gefüllt ist. In Schritt 250 wird ein zweites Untersuchungsmedium bereitgestellt. Typischerweise ist das zweite Untersuchungsmedium das Fluid des ersten Untersuchungsmediums ohne die Partikel der Suspension des ersten Untersuchungsmediums. Die Partikelgrößenverteilung des ersten Untersuchungsmediums am Ausgang des Untersuchungsobjekts wird zu Beginn des Schritts 250 gemessen und bildet einen Referenzwert. Die Masse wird vor dem Durchleiten des zweiten Untersuchungsmediums als Referenzwert gemessen. Im Schritt 260 wird das zweite Untersuchungsmedium durch das Untersuchungsobjekt durchgeleitet. Im Schritt 270 werden die Partikelgrößenverteilung und die Änderung der Partikelgrößenverteilung nach dem Durchleiten des zweiten Untersuchungsmediums durch das Untersuchungsobjekt bestimmt. In Schritt 280 wird geprüft, ob eine Änderung der Partikelgrößenverteilung oder eine Änderung der Gesamtmasse auftritt. Sofern diese beobachtet wird, wird das Durchleiten des zweiten Untersuchungsmediums durch das Untersuchungsobjekt fortgesetzt. Dabei werden die im Schritt 270 bzw. 280 gemessenen Werte als neue Referenzwerte genommen. Sofern das Verfahren konvergiert und keine Änderung der Partikelgrößenverteilung oder der Gesamtmasse mehr festgestellt werden, ist das Verfahren konvergiert und in Schritt 285 wird der Reinigungsgrad der Anlage als "festgestellt" betrachtet. Damit ist das Verfahren beendet.

Es gilt also: die vorgestellte Erfindung beruht auf der Betrachtung eines Apparats oder einer Rohrleitung als Trennvorrichtung im Hinblick auf eine Partikelsuspension. Entsprechend wird eine abgesicherte Beurteilung des Hygienestatus einer Anlage bereitgestellt. Es kann somit ein Kontrollinstrument zur Beurteilung des Verschmutzungs- und Reinigungsverhaltens einer Anlage bereitgestellt werden. Durch Tests einer Anlage, beispielsweise einer Kurzzeiterhitzungsanlage für Milchprodukte, kann eine Aussage getroffen werden, wie schwer oder leicht die Anlage zu verschmutzen ist. Entsprechend können Aussagen über Standzeiten getroffen werden; darauf aufbauend kann ein Reinigungsprogramm formuliert werden. Entsprechend kann eine beschriebene Detektionsvorrichtung mit optischen Signalen für Getränkesuspensionen bereitgestellt werden. Grundlage hierfür kann die Detektion der Partikelgrößenverteilungsänderung einer bestimmten im Produkt vorhandenen Substanz sein, beispielsweise Fasern oder Fruchtbestandteile in einem Saft.

Zusammenfassend kann gesagt werden, dass es bisher keinen mikrobiologischen Standard zur Hygienestatusbeurteilung gibt. Ein solcher Standard ließe sich jedoch, sofern er existieren sollte, in das entsprechende Verfahren integrieren. Das entsprechende mechanisch verfahrenstechnische Verfahren ist robust und besitzt eine hohe Reproduzierbarkeit. Die Handhabung, insbesondere mit einer Sand-Wasser-Suspension, insbesondere Quarzsand-Wasser, ist empfehlenswert, weil Quarzsand und Wasser sich nicht miteinander umsetzen. Ferner ist eine derartige Suspension temperaturbeständig. Sie ist ebenso lagerbeständig. Die suspendierten Quarzpartikel sind leicht von organischen Partikeln abzutrennen. Und die Suspension wird nicht durch Mikroorganismen umgesetzt. Eine solche Suspension ist ferner ungefährlich für Mensch und Maschine und aufgrund der hohen Verfügbarkeit von beiden Bestandteilen ist eine derartige Suspension sehr preiswert herzustellen. Die entsprechenden Ergebnisse der Anlage stehen praktisch sofort nach Test einer Anlage zur Verfügung, es gibt keinen Aufwand für Bebrütungs- oder Analysezeiten. Der derartige apparative Aufwand ist gering, und ermöglicht, sich verschiedene Anlagen und verschiedene Produkte in Anlagen miteinander zu vergleichen.

Es versteht sich, dass in den zuvor beschriebenen Ausführungsbeispielen genannten Merkmale sich nicht nur auf die speziell in den Figuren gezeigten Kombinationen beschränken, sondern auch in beliebigen anderen Kombinationen möglich sein können.

**Patentansprüche**

1. Verfahren zum Bestimmen des Verschmutzungsgrades eines Untersuchungsobjekts (2) wie etwa einer Anlage, insbesondere einer Anlage zur Abfüllung flüssiger Lebensmittel, oder eines Bauteils oder Teilabschnitts einer Anlage, insbesondere einem Rohr, **gekennzeichnet durch**
Messen einer Eigenschaftsverteilung eines ersten Untersuchungsmediums vor und nach dem Durchleiten **durch** das Untersuchungsobjekt (2).

2. Verfahren nach Anspruch 1, wobei das erste Untersuchungsmedium ein erstes Fluid umfasst.

3. Verfahren nach Anspruch 2, wobei das erste Fluid ein flüssiges Lebensmittel umfasst.

4. Verfahren nach Anspruch 2, wobei das erste Fluid Wasser umfasst.

**5.** Verfahren nach einem der Ansprüche 2 - 4, wobei das erste Untersuchungsmedium einen Feststoff, insbesondere eine Mischung wie etwa eine Suspension des ersten Fluids und des Feststoffs, insbesondere Partikel, umfasst.

**6.** Verfahren nach Anspruch 5, wobei die Partikel solche Feststoffe umfassen, die im Lebensmittel enthalten sind oder die dem Lebensmittel zugesetzt worden sind.

**7.** Verfahren nach einem der Ansprüche 5 oder 6, wobei die Partikel sandartige Partikel, insbesondere Quarzsand, umfassen.

**8.** Verfahren nach einem der Ansprüche 5 - 7, wobei die Eigenschaftsverteilung des ersten Untersuchungsmediums die Partikelgrößenverteilung und/oder die Änderung der Partikelgrößenverteilung vor und nach dem Durchleiten durch das Untersuchungsobjekt (2) umfasst.

**9.** Verfahren nach einem der Ansprüche 5 - 8, wobei beim Messen der Eigenschaftsverteilung des ersten Untersuchungsmediums die Massenänderung, insbesondere der prozentuale Massenverlust oder -zuwachs, bestimmt wird.

**10.** Verfahren nach einem der Ansprüche 1 - 9, wobei nach dem Bestimmen des Verschmutzungsgrades bei mit dem ersten Untersuchungsmedium gefüllten Untersuchungsobjekt das Reinigungsverhaltens des Untersuchungsobjekts (2) bestimmt wird, wobei eine Eigenschaftsverteilung eines zweiten Untersuchungsmediums vor und nach dem Durchleiten durch das Untersuchungsobjekt (2) gemessen wird, und
die Eigenschaftsverteilung des zweiten Untersuchungsmediums nach dem Durchleiten mit der Eigenschaftsverteilung des ersten Untersuchungsmediums nach dem Durchleiten verglichen wird.

**11.** Verfahren nach Anspruch 10, wobei das zweite Untersuchungsmedium ein zweites Fluid umfasst, insbesondere wobei das zweite Fluid dem ersten Fluid entspricht.

**12.** Verfahren nach einem der Ansprüche 10 - 11, wobei die Eigenschaftsverteilung des zweiten Untersuchungsmediums die Partikelgrößenverteilung und/oder die Änderung der Partikelgrößenverteilung vor und nach dem Durchleiten umfasst.

**13.** Verfahren nach Anspruch 12, wobei beim Messen der Eigenschaftsverteilung des zweiten Untersuchungsmediums die Massenänderung, insbesondere der prozentuale Massenverlust oder -zuwachs, bestimmt wird.

**14.** Verfahren nach einem der Ansprüche 1 - 13, wobei das Messen mittels eines Laserbeugungsspektrometers erfolgt.

**15.** Verfahren nach einem der Ansprüche 8 oder 12, wobei das Messen der Partikelgrößenverteilung und/oder der Änderung der Partikelgrößenverteilung in vordefinierten Zeitabständen erfolgt oder in kontinuierlicher Form erfolgt.

**16.** Verfahren nach einem der Ansprüche 9 oder 13, wobei das Messen der Massenänderung wenigstens zu Beginn und zum Ende des Messens erfolgt.

**17.** Vorrichtung zum Durchführen des Verfahrens nach einem der Ansprüche 1 - 16, mit einem Aufgabe- und einem Ausgabebehälter (1, 2);
mit einer Fördervorrichtung zum Entnehmen des ersten oder des zweiten Untersuchungsmediums aus dem Aufgabebehälter (1) und zum Durchleiten des ersten oder des zweiten Untersuchungsmediums durch das Untersuchungsobjekt (2) und zum Auffangen des ersten oder des zweiten Untersuchungsmediums im Ausgabebehälter (2); und mit einer Detektionsvorrichtung (4B, 4A, 5) zum Bestimmen der Eigenschaftsverteilung des ersten oder des zweiten Untersuchungsmediums.

**18.** Vorrichtung nach Anspruch 15, mit einer Homogenisierungsvorrichtung (6A, 6B) im Aufgabe und/oder Ausgabebehälter (1, 2).

**19.** Vorrichtung nach einem der Ansprüche 15 oder 16, mit Messvorrichtungen zur Messung von Temperatur, Druck, Partikelgrößen von Partikeln in dem ersten und/oder zweiten Untersuchungsmedium, sowie weiterer Eigenschaftsverteilungen in den Aufgabe- und/oder Ausgabebehältern (1,2) und im Untersuchungsobjekt (2).

**20.** Vorrichtung nach einem der Ansprüche 15 - 17, mit einem Messsystem, welches Laserbeugungsspektroskopie zur Bestimmung der Partikelgrößenverteilungen und zur Massenbestimmung einsetzt.

FIG. 1A

FIG. 1B

FIG. 2B

FIG. 2A

FIG. 3

FIG. 4

Bereitstellen des ersten Untersuchungsmediums, Messen von Partikelgrößenverteilung, Masse vor dem Durchleiten durch das Untersuchungsobjekt —210

Durchleiten des ersten Untersuchungsmediums durch das Untersuchungsobjekt —220

Messen von Partikelgrößenverteilung, Änderung der Partikelgrößenverteilung, Masse nach dem Durchleiten durch Untersuchungsobjekt —230

240
Änderung Partikelgrößenverteilung? Änderung Gesamtmasse?

ja

nein

Verschmutzungsgrad der Anlage festgestellt, Objekt ist mit erstem Untersuchungsmedium gefüllt —245

Bereitstellen des zweiten Untersuchungsmediums, Messen von Partikelgrößenverteilung des ersten Untersuchungsmediums am Ausgang des Untersuchungsobjekts, Masse vor dem Durchleiten des zweiten Untersuchungsmediums durch das Untersuchungsobjekts —250

Durchleiten des zweiten Untersuchungsmediums durch das Untersuchungsobjekt —260

Messen von Partikelgrößenverteilung, Änderung der Partikelgrößenverteilung, Masse nach dem Durchleiten durch Untersuchungsobjekt —270

280
Änderung Partikelgrößenverteilung? Änderung Gesamtmasse?

ja

Reinigungsgrad der Anlage festgestellt —285

FIG. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102009009426 A1
- WO 9800694 A